(19) **Europäisches Patentamt European Patent Office Office européen des brevets**

(11) **EP 4 679 447 A1**

(12) **EUROPEAN PATENT APPLICATION**

(43) Date of publication:
**14.01.2026 Bulletin 2026/03**

(21) Application number: **25188882.2**

(22) Date of filing: **10.07.2025**

(51) International Patent Classification (IPC):
**G16H 30/40** (2018.01)     **G16H 50/20** (2018.01)

(52) Cooperative Patent Classification (CPC):
**G16H 50/20; G16H 30/40;** A61B 5/0088;
G06T 7/0012; G06V 2201/03; G16H 50/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **12.07.2024   US 202463670318 P**

(71) Applicant: **Hamad Bin Khalifa University Doha (QA)**

(72) Inventors:
• **El-Shrif, Mohamed M.**
  **Doha (QA)**
• **Isufaj, Keivin**
  **Doha (QA)**
• **Ba Hattab, Raidan Abdullah Saeed**
  **Doha (QA)**
• **Shaban, Khaled Bashir**
  **Doha (QA)**
• **Tamimi, Faleh**
  **Doha (QA)**

(74) Representative: **K&L Gates LLP**
  **Friedrichstraße 110 A**
  **10117 Berlin (DE)**

(54) **HUMAN-ARTIFICIAL INTELLIGENCE COLLABORATIVE PLATFORM FOR ORAL CANCER LESION DIAGNOSIS**

(57)     Example systems, methods, and apparatus are disclosed herein for oral cancer lesion diagnosis. An image capture device, such as a camera, can capture images and videos of a patient's mouth during consultations and providing instantaneous feedback on potential lesions. The image capture device is in communication with a collaborative platform. The platform includes an artificial intelligence feature having a machine learning model. The platform further includes a human interface configured to receive user input.

FIG. 3

**Description**

CROSS-REFERENCE TO RELATED APPLICATION

The present disclosure claims priority to U.S. Provisional Patent Application 63/670,318 having a filing date of July 12, 2024, the entirety of which is incorporated herein.

BACKGROUND

Oral cancer (OC) represents a critical global health concern with high mortality rates, accounting for over one-third of a million new cases and nearly two-hundred thousand deaths in 2020. OC survival rates are alarmingly low, at less than 50%, and highly associated with the stage at which the cancer is diagnosed. In comparison to other cancer types, OC patients face even lower survival rates, such as those seen in breast and prostate cancers. This emphasizes the critical importance of early detection, as any delay in diagnosing OC lesions dramatically increases mortality rates.

Detecting OC lesions at early stages poses a significant challenge for several reasons. First, novice dentists primarily rely on visual examination using a penlight, and when suspicious lesions are identified, invasive procedures like biopsies are required. Second, primary healthcare centers and general practitioners often lack specialized training OC diagnosis, contributing to the difficulty of early detection. Third, existing OC detection methods, including vital dyes and cytology evaluation techniques, suffer from issues of low specificity, leading to either missed early-stage lesions (high false-negative rates) or the misclassification of non-cancerous regions as lesions (high false-positive rates).

Recent studies have emphasized the positive impact of regular clinical screening programs on OC incidence and mortality rates. Deploying such screening programs at a national level is a proposed solution for early detection. However, in practice, only a few countries have implemented national OC screening programs, and limited resources in low and middle-income countries pose challenges for regular screening.

Given these challenges, there is a need for noninvasive, user-friendly, and computationally efficient tools that can accurately detect OC lesions at an early stage and differentiate between different OC types.

SUMMARY

Recent advances in artificial intelligence (AI) and machine learning (ML) offer promising avenues for the development of such tools. These clinical decision support platforms harness AI techniques to provide initial or secondary diagnoses, enhancing the accuracy and efficiency of clinician decision-making.

Clinical decision support platforms can generally be categorized into rule-based and ML-based platforms. While rule-based platforms are interpretable and flexible, they struggle to create generalizable rules for complex decision support tasks. In contrast, ML-based platforms use advanced ML architectures to automatically extract features for decision support tasks, with deep learning (DL) being a prominent focus in recent research. Although ML-based platforms demonstrate promising performance in terms of accuracy and computational efficiency, their integration into real-world settings is hindered by a lack of human-centered design.

Example systems, methods, and apparatus are disclosed herein for a human-artificial intelligence collaborative platform for oral cancer lesion diagnosis.

In light of the disclosure herein, and without limiting the scope of the invention in any way, in a first aspect of the present disclosure, which may be combined with any other aspect listed herein unless specified otherwise, a system for oral cancer lesion diagnosis includes an image capture device configured to capture at least one image of a mouth and a platform. The platform includes an artificial intelligence feature including a machine learning model configured to detect a potential oral cancer lesion based on the at least one image of the mouth and a user interface configured to receive user input.

In another aspect of the present disclosure, which may be combined with any other aspect listed herein unless specified otherwise, the image capture device is further configured to capture at least one video of the mouth.

In another aspect of the present disclosure, which may be combined with any other aspect listed herein unless specified otherwise, the user interface is configured to allow a user to manually mark and annotate the at least one image of the mouth.

In another aspect of the present disclosure, which may be combined with any other aspect listed herein unless specified otherwise, the user interface includes at least one user role defining user access privileges to the platform.

In another aspect of the present disclosure, which may be combined with any other aspect listed herein unless specified otherwise, the user roles include at least one of administrator, dentist, reviewer, data scientist, guest, and student.

In another aspect of the present disclosure, which may be combined with any other aspect listed herein unless specified otherwise, the platform is configured to operate in an online mode and an offline mode.

# EP 4 679 447 A1

In another aspect of the present disclosure, which may be combined with any other aspect listed herein unless specified otherwise, the at least one image of the mouth is processed by the machine learning model.

In another aspect of the present disclosure, which may be combined with any other aspect listed herein unless specified otherwise, wherein upon detecting a potential oral cancer lesion, the artificial intelligence feature notifies the user.

In another aspect of the present disclosure, which may be combined with any other aspect listed herein unless specified otherwise, wherein upon detecting a potential oral cancer lesion, the artificial intelligence feature sends the at least one image of the mouth to a specialist for review.

In another aspect of the present disclosure, which may be combined with any other aspect listed herein unless specified otherwise, wherein the artificial intelligence feature further includes a segmentation model that processes the at least one image of the mouth and highlights a potential oral cancer lesion.

In another aspect of the present disclosure, which may be combined with any other aspect listed herein unless specified otherwise, wherein the artificial intelligence feature further includes a classification model.

In another aspect of the present disclosure, which may be combined with any other aspect listed herein unless specified otherwise, wherein the artificial intelligence feature is configured to perform pre-processing and transformation techniques including at least one of resizing the at least one image of the mouth, augmenting data associated with the at least one image of the mouth, and normalizing pixel values of the at least one image of the mouth.

In another aspect of the present disclosure, which may be combined with any other aspect listed herein unless specified otherwise, a method for diagnosing an oral cancer lesion includes providing an image capture device configured to capture at least one image of a mouth; providing a platform, the platform including an artificial intelligence feature including a machine learning model; and detecting a potential oral cancer lesion via the machine learning model based on the at least one image of the mouth.

In another aspect of the present disclosure, which may be combined with any other aspect listed herein unless specified otherwise, the platform includes a user interface configured to receive user input.

In another aspect of the present disclosure, which may be combined with any other aspect listed herein unless specified otherwise, the method further includes processing, via the machine learning model, the at least one image of the mouth.

In another aspect of the present disclosure, which may be combined with any other aspect listed herein unless specified otherwise, the method further includes upon detecting a potential oral cancer lesion, notifying a user.

In another aspect of the present disclosure, which may be combined with any other aspect listed herein unless specified otherwise, the method further includes upon detecting a potential oral cancer lesion, sending the at least one image of the mouth to a specialist for review.

In another aspect of the present disclosure, which may be combined with any other aspect listed herein unless specified otherwise, the method further includes processing the at least one image of the mouth and highlighting a potential oral cancer lesion.

In another aspect of the present disclosure, which may be combined with any other aspect listed herein unless specified otherwise, the artificial intelligence feature further includes a classification model.

In another aspect of the present disclosure, which may be combined with any other aspect listed herein unless specified otherwise, the method further comprises performing pre-processing and transformation techniques including at least one of resizing the at least one image of the mouth, augmenting data associated with the at least one image of the mouth, and normalizing pixel values of the at least one image of the mouth.

In light of the present disclosure and the above aspects, it is therefore an advantage of the present disclosure to provide users with a human-artificial intelligence collaborative platform for oral cancer lesion diagnosis and method for using a human-artificial intelligence collaborative platform for oral cancer lesion diagnosis.

It is a further advantage to provide pre-trained ML models to segment oral lesion areas.

Additional features and advantages are described in, and will be apparent from, the following Detailed Description. The features and advantages described herein are not all-inclusive and, in particular, many additional features and advantages will be apparent to one of ordinary skill in the art in view of the figures and description. In addition, any particular embodiment does not have to have all of the advantages listed herein and it is expressly contemplated to claim individual advantageous embodiments separately. Moreover, it should be noted that the language used in the specification has been selected principally for readability and instructional purposes, and not to limit the scope of the inventive subject matter.

## BRIEF DESCRIPTION OF THE DRAWINGS

Figure 1A illustrates a healthy oral lesion, according to an example embodiment of the present disclosure.

Figure 1B illustrates tongue cheek chewing lesion, according to an example embodiment of the present disclosure.

Figure 1C illustrates a geographic tongue lesion, according to an example embodiment of the present disclosure.

Figure 2 illustrates a Phase 1 system architecture diagram, according to an example embodiment of the present disclosure.

Figure 3 illustrates a Phase 2 system architecture diagram, according to an example embodiment of the present disclosure.

Figure 4 illustrates a view of an interface that shows when a new patient is assigned to a doctor or annotator, both receive a notification and a text message highlighting the patient's ID, according to an example embodiment of the present disclosure.

Figure 5 illustrates a view of an interface that shows the admin viewing the segmentation page that shows the machine learning segmentation result by the back-end model, according to an example embodiment of the present disclosure.

Figure 6 illustrates a view of an interface that shows the view for a nurse adding a new patient, according to an example embodiment of the present disclosure.

Figure 7 illustrates a view of an interface for doctors/annotators with an option to highlight lesions using a rectangular shape or a multi-point polygon, according to an example embodiment of the present disclosure.

Figure 8 illustrates a view of an interface of a reviewer, according to an example embodiment of the present disclosure.

Figure 9 illustrates a view of an interface of an IT/Engineer highlighting the system logs and any errors that are occurring, according to an example embodiment of the present disclosure.

Figure 10 illustrates a view of an interface that shows a patient report form from a student's perspective, according to an example embodiment of the present disclosure.

Figure 11 illustrates a view of an interface that shows stored images alongside segmentation and classification results, according to an example embodiment of the present disclosure.

Figure 12 illustrates a view of an interface that shows system workflow, according to an example embodiment of the present disclosure.

Figure 13 illustrates a view of an interface that shows selected results of the segmented images and the visual output of the segmentation model, reflecting the model's ability to detect multiple lesion instances, according to an example embodiment of the present disclosure.

Figure 14 illustrates an Intersection over Union (IoU) of two masks, according to an example embodiment of the present disclosure.

## DETAILED DESCRIPTION

Methods, systems, and apparatus are disclosed herein for a human-artificial intelligence collaborative platform for oral cancer lesion diagnosis.

While the example methods, apparatus, and systems are disclosed herein for a human-artificial intelligence collaborative platform for oral cancer lesion diagnosis, it should be appreciated that the methods, apparatus, and systems may be operable for other applications.

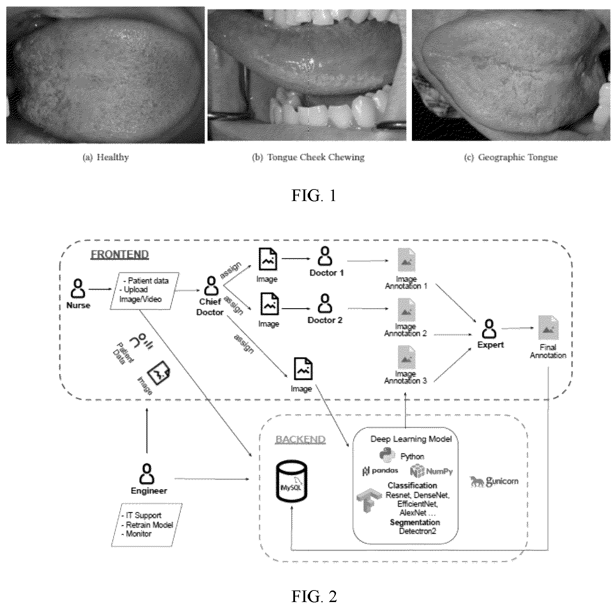

At early stages of oral cancer (OC) lesion growth, dentists often struggle to distinguish OC lesions as they can resemble clinically normal "healthy" regions. For example, Figure 1A illustrates a healthy mouth including no lesions. Figure 1B illustrates a mouth having a tongue cheek chewing OC lesion. Figure 1C illustrates a mouth having a geographic tongue lesion. Because of the similar appearance, dentists may struggle with the detection of OC lesions, particularly at early stages.

The proposed technology is a human-AI collaborative platform for OC lesion diagnosis. The proposed technology integrates advanced ML models with human interaction, enabling self-examination and initial screening for regular individuals. It also offers automatic feedback to general practitioners who may lack the expertise to identify OC lesions during regular patient visits. Additionally, healthcare specialists can intervene in the decision-making process, assessing and correcting lesion areas and altering subsequent interventions and treatment plans. The proposed technology leverages a substantial existing annotated dataset created by oral medicine specialists with over 30 years of experience, making it a valuable tool for diagnosing OC lesions. The platform promotes user engagement with ML models, fostering human-AI collaborative decision-making.

An image capture device, such as a camera, can capture images and videos of a patient's mouth during consultations and providing instantaneous feedback on potential lesions. The image capture device is in communication with a collaborative platform. The platform includes an artificial intelligence feature having a machine learning model. The platform further includes a human interface configured to receive user input.

The platform includes two phases. In Phase I, the ground truth creation phase, a user-friendly interface is provided for annotators, primarily dentists, to manually mark and annotate input images, creating a ground truth labeled corpus. Figure 2 illustrates Phase I. To enhance accuracy, multiple experts review the same images, with a specialist confirming final annotations. After establishing this ground truth, the data is used to train DL models, preparing them for future OC lesion segmentation and classification tasks.

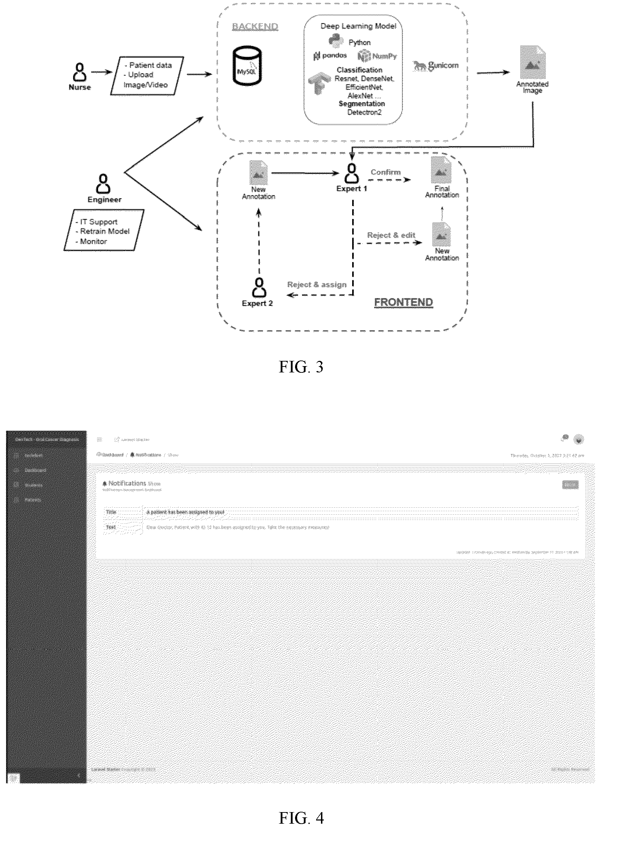

Figure 3 illustrates Phase II, the practical application phase, of the platform. Phase II illustrates the practical application of the platform. During a patient's visit, images or live video streams of the patient's mouth are captured. These images are promptly processed by the platform, leveraging pretrained models to provide instantaneous AI feedback, including both segmentation and classification of potential OC lesions. Dentists review this AI-generated outcomes, and if uncertainties arise, they forward the images to an OC lesion specialist. This expert has the authority to validate, rectify, or specify lesion details. Images fine-tuned by the specialist are then harnessed by data scientists to further refine and train the deep learning models, ensuring continuous improvement of the system's accuracy.

The platform can define user roles including roles for administrator/chief doctor, nurse/doctor's assistant, dentist/doctor/annotator, specialist/reviewer, data scientist/systems support engineer, guest/self-screening user, and student. It will be appreciated that in other embodiments of the platform, additional user roles may be added or omitted.

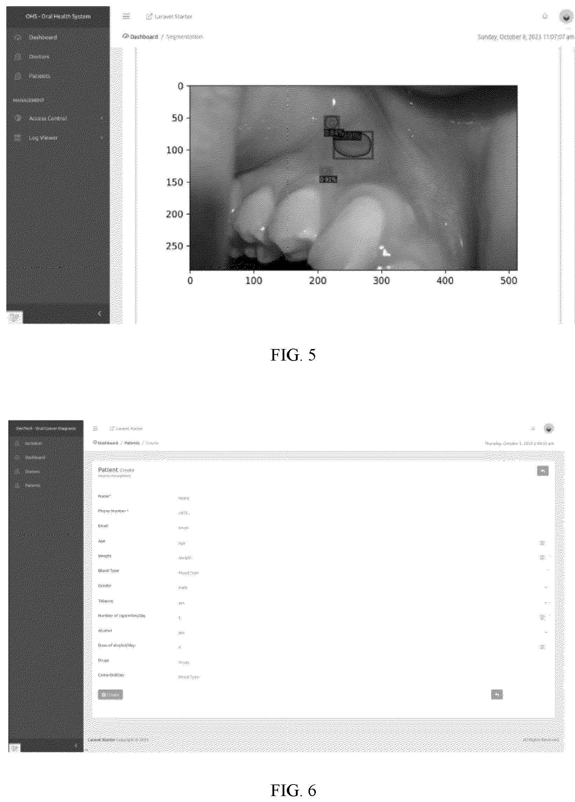

Administrator/Chief Doctor: The chief doctor has full access to the entire platform with full privileges. They can create new user accounts, such as nurse/doctor's assistant, dentist, and specialist, each with specific roles and permissions. The administrator can view patient records, assign patient images to annotators (see Figures 4 and 5), and manage user permissions. They are responsible for overseeing the system and configuring user views.

Nurse/Doctor's Assistant: Nurses or doctor's assistants can add new patients to the platform, enter patient metadata, and upload images. They lack the permissions to perform image annotations, or remove patient records. Figure 6 illustrates the nurse user interface for adding a new patient. The nurse is prompted to complete a form capturing essential patient details. This includes the patient's name, phone number, age, gender and lifestyle indicators such as smoking and alcohol consumption, among other information.

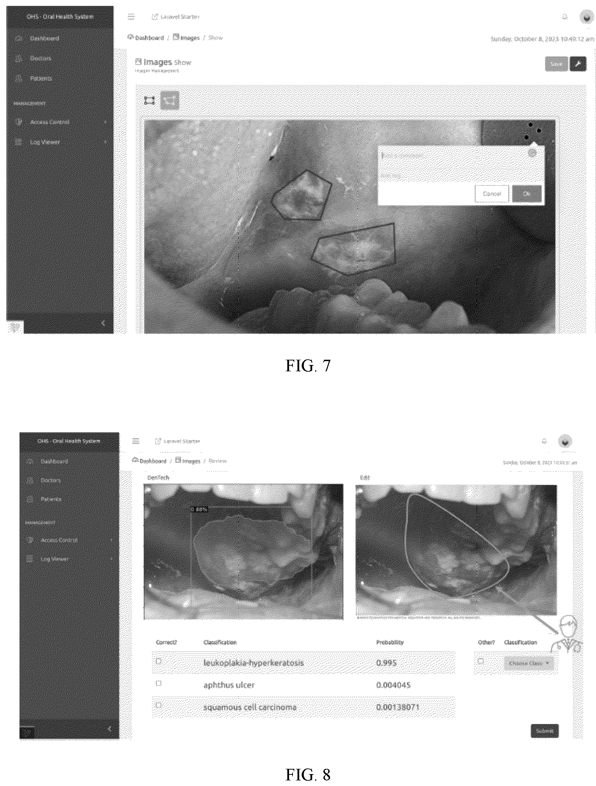

Dentist/Doctor/Annotator: Dentists or novice doctors can access patient images assigned to them, annotate images manually or use annotation tools. They play a key role in the ground truth creation process. Figure 7 illustrates a doctor's view when annotating the images, adding either rectangular shapes or multi-point polygon shapes to highlight the OC lesion areas. Additionally, doctors can provide supplementary comments as needed. For example, the dentist can write a comment and/or add a tag to each annotation. Once this task is done, the annotation can be saved, and the status of the image will be updated for the administrator to be assigned to a specialist to review it and make the final decision.

Specialist/Reviewer: Specialists or reviewers are experts in dental medicine/pathology. They review annotations made by dentists and resolve annotation conflicts. They ensure the accuracy of annotations and contribute to the ground truth data. Figure 8 shows the interface that is available to the reviewers, where they are able to choose which annotation is correct, and can even edit the annotations themselves. In this figure, the reviewer is shown editing an annotation by a specialist.

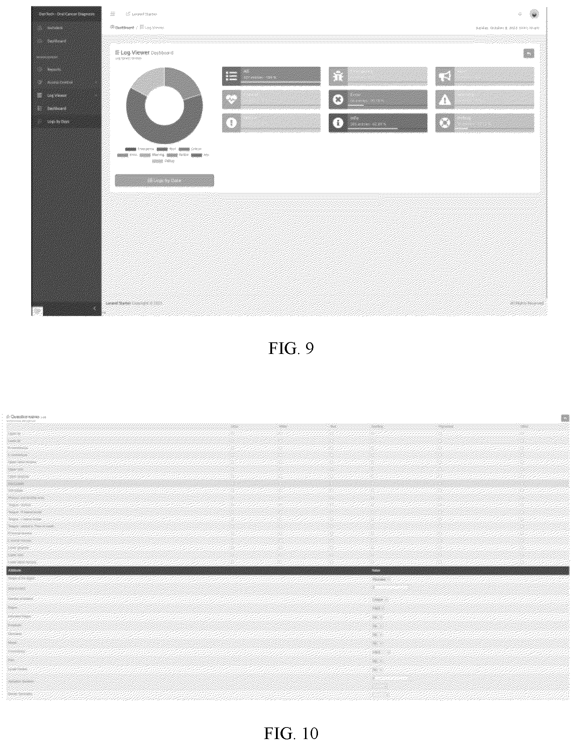

Data Scientist/Systems Support Engineer: Data scientists or systems support engineers are responsible for system maintenance, user account management, model updates, and integrating new modules into the platform. A data scientist is able to view and edit each user's permissions, but not patient's data. Also, they can view the users' logs and export different statistics about the system, which could be reported to the upper management. Figure 9 depicts the log view dashboard, showing the system logs and any errors that are occurring. By default, every 500 new images, or once a month, the data scientist receives a notification to update/re-train the ML models. In addition, this dashboard can be used to track the historical graphs about the performance of the system in terms of accuracy and computational efficiency.

Guest/Self-Screening User: Guests or self-screening users can register, log in, and use the platform for self-examination without requiring a doctor's intervention. So, the guest user can take images/videos for his/her mouth and view the AI predictions. In case, the AI shows any positive results; he/she can share the images with a dentist, or specialist for further investigation.

Student: Students with the credentials from the chief doctor can register new patients, add patient data, and contribute to lesion reports (Figure 10), which cover aspects such as size, presence of pain, and description, facilitating an interactive and comprehensive review.

Further, the proposed technology's GUI offers both online and offline modes for the segmentation and classification of OC lesion areas. In the online mode, nurses or dentist assistants can capture in real-time images using an intraoral camera, initiating live recording (i.e., videos). Frames are selected at intervals for processing by pretrained models. Results are displayed for immediate use, and images with suspected OC lesions can be sent to specialists for review and model enhancement. In the offline mode, authorized users can upload and process images. The system treats these uploads similarly to the online mode, providing results and allowing for further specialist review and model improvement.

Oral cancer detection within the platform is a meticulous two-step process. Initially, images undergo segmentation to pinpoint the lesion or region of interest (ROI). Proposed technology's dedicated segmentation model that automatically processes images, highlighting potential OC lesions with confidence levels. The back-end model continuously evolves with updated annotated images for improved accuracy. A visual representation of this process is illustrated in Figure 5.

Following segmentation, the ROI undergoes classification within a classification model. The proposed technology utilizes multiple deep learning models for this purpose. When an image or video is introduced, several models process it simultaneously, presenting the most accurate predictions to users. Results include potential cancer types and confidence levels, facilitating further specialist validation. A visual representation of this classification process can be found in Figure 8.

The proposed technology is built on the Laravel framework for PHP, using the Model-View-Controller (MVC) architecture combined with the RESTful API design. User requests and image uploads are securely stored in a back-end MySQL database. Nginx acts as a reverse proxy and load balancer, while Flask, a Python web framework, manages image processing, segmentation, and classification. The back-end ensures efficient model loading, secure API requests, and data record maintenance, enabling specialists to validate predictions and improve model performance.

The proposed technology is a deep learning component that can automatically detect OC lesions, delineate the lesion area, and predict the most probable cancer type. The proposed technology deep learning model is designed to perform segmentation and classification on natural OC input images and video streams.

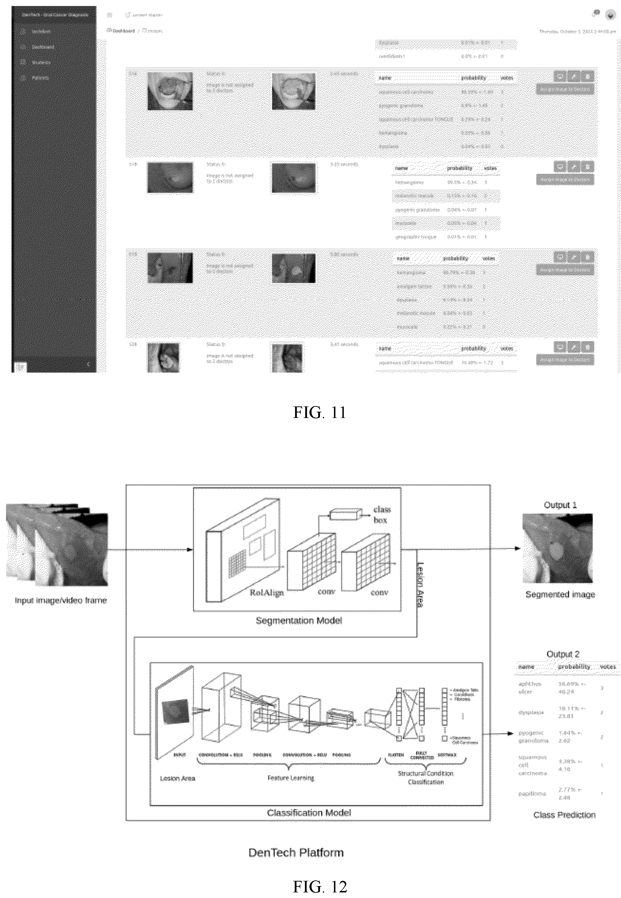

As illustrated in Figure 12, the approach begins with the development of a deep learning model skilled in object detection and instance segmentation. Following this, the proposed technology crops the images according to the detected bounding boxes. These cropped images then serve as inputs for a specialized deep learning classification model, which is designed to identify if the image showcases a lesion and, if so, determine its stage and type.

In an exemplary embodiment of the present disclosure, to train the machine learning model, 3,271 clinical oral photographs from 5 hospitals/universities in Canada, Spain, Qatar, and Pakistan between 2000 and 2023 were retrospectively collected. 2,290 photographs (training dataset) were randomly selected to develop the algorithm/framework and used the remaining 981 photographs (testing dataset) for validation purposes. All biopsy-proven photographs were included and normal controls by performing image quality control to remove various artifacts. The dataset comprises photographs representing healthy oral conditions as well as various oral lesions, including Amalgam Tattoo, Aphthous Ulcer, Candidiasis, Fibroma, Geographic Tongue, Hemangioma, Leukoplakia - Hyperkeratosis, Lichen Planus, Melanotic Macule, Mucocele, Papilloma, Pyogenic Granuloma, Squamous cell Carcinoma, and Tongue Cheek Chewing, along with different lesion classifications based on the potential to spread to other parts of the body, including benign, premalignant, and malignant.

The diagnoses of these lesions were made by oral pathology specialists with 30 years of experience, and malignant lesions were confirmed through histopathology diagnosis. To ensure privacy and accuracy, the regions of interest in each image were masked by two specialized dentists with 15 years of clinical experience using the LabelMe annotation tool, ensuring that each image has its corresponding annotation. The corresponding diagnosis reports were used as the gold standard to develop and validate the deep learning algorithm.

The dataset presents various challenges, including different image sizes, class imbalances, different angles of image shots, and variations in brightness. To address these challenges, the proposed technology employs several data pre-processing and transformation techniques:

Resizing Input Images: Adjustment of the image sizes matches the requirements of different deep learning models. Calculation of the new dimensions of an input image based on its aspect ratio, ensuring it aligns with the optimal input size for each model. Since the input images and video frames are taken through different devices and mediums, the proposed technology adjusts images with width w, height of $h$, ratio of $r = w/h$, and the required aspect ratio be $r = w_{new}/h_{new}$. The proposed technology defines the new dimensions of an input image as follows:

$$\begin{cases} w_{new} = w, h_{new} = \frac{w_{new}}{r}, \ if \ r^- < r \\ w_{new} = h * r, h_{new} = h \, , \ if \ r^- > r \\ \quad w_{new} = w, h_{new} = h \, , \ if \ r^- = r \end{cases}$$

Data Augmentation: To mitigate class imbalances and enhance model robustness, the proposed technology applies data augmentation techniques. These techniques include cropping, rotating, flipping, and introducing slight variations in brightness and contrast, reflecting real-world scenarios.

Normalization: the proposed technology applies the pixel values of the images to a scale of [0,1] and normalize them using the standard ImageNet normalization values [24], reducing data skewness and facilitating model training. The

$$output[channel] = \frac{input[channel] - mean[channel]}{std[channel]}$$

normalization is performed as following:                                                                                                , where the *mean* and *std* are the mean and the standard deviation based on the values of ImageNet database.

Segmentation of OC lesions presents several challenges, including variations in mask areas, distinct color differences between lesions and healthy tissue, and a lack of standardized image conditions. The proposed technology prioritizes a class-agnostic segmentation to accurately delineate lesion boundaries, leaving the specific lesion type classification to dedicated models. The segmentation model combines binary classification, a Region Proposal Network, and a Region of Interest Alignment Layer. The loss function includes terms for classification, bounding box prediction, and mask prediction. The segmentation goal is to minimize a loss function, which is now composed of three subsets, as follows: $L_{seg} = L_{class} + L_{box} + L_{mask}$, where $L_{class}$ is the loss of the classification (background or lesion), $L_{box}$ measures the difference between the predicted bounding box and the true bounding box, while $L_{mask}$ is another entropy loss function that penalizes pixels of the image that are incorrectly identified as inside/outside the predicted mask compared to the ground truth.

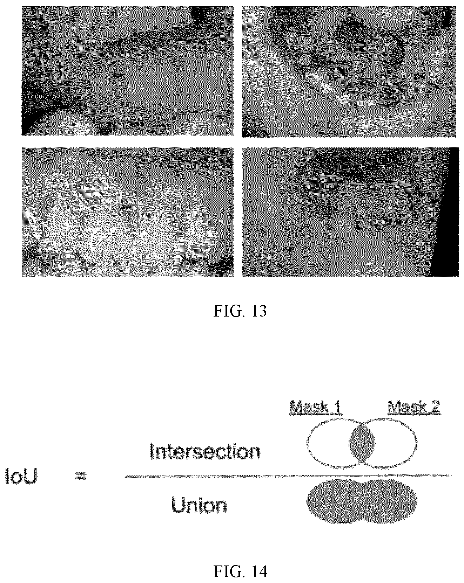

Segmentation focuses on determining the efficacy and breadth of the predicted mask or annotated region of a potential OC lesion. Accuracy is assessed by comparing the pixels of the predicting mask with those of the ground truth obtained in Phase I. The primary metric for the segmentation model is the Average Precision (AP), which computes the area beneath the precision-recall curve. The predicted masks are evaluated against manually annotated ones using the Intersection over Union (IoU) metric, illustrated in Figure 14, which measures the overlap between the two masks as a percentage of their cumulative area. IoU is assessed at ten distinct thresholds, ranging from 0.5 to 0.95, incremented by 0.05 units and calculated as depicted by the following equations:

$$ AP_{CLASS} = \frac{1}{\#threshold} \sum_{IoU \in thresholds} AP[class, IoU] $$

where $AP[class, IoU] = \int_0^1 pr(r)dr$. To further evaluate the precision at specific IoU thresholds, $AP_{50}$ and $AP_{75}$ were reported for thresholds of 0.5 and 0.75, respectively. Additionally, $AP_L$ was calculated, representing the average precision for large objects where the area is greater than $96^2$ units.

The accuracy, an essential metric in classification, is calculated by the ratio of true positives (TP) and true negatives (TN) to the overall number of samples. It provides a holistic measure of the model's performance. Mathema-

$$ Accuracy = \frac{TP+TN}{TP+TN+FP+FN} $$

tically, it is defined as: . Here, TP and TN represent correctly identified positive and negatives samples, respectively, while FP and FN denote falsely identified positives and negatives.

The proposed technology performs classification tasks for lesion stage and lesion type detection. The proposed technology adopts transfer learning on pre-trained models that have already been trained on large-scale datasets such as ImageNet to leverage their knowledge of visual features. Classification is not performed on raw images but on cropped regions of interest corresponding to the lesions. The proposed technology fine-tunes the pre-trained models on our custom dataset, keeping the earlier layers frozen and the proposed technology trains the last layers from scratch. This approach adapts the models to our dataset's specific classes and features while benefiting from the pre-trained model's initial performance. The existing knowledge is stored in millions of parameters that allows us to significantly accelerate the prediction classification capabilities for tasks such as oral lesion classification.

The proposed technology models are designed for continuous improvement. User interactions with the platform lead to routine scrutiny of model predictions by expert specialists. These medical professionals validate the predictions and provide feedback, which is used for model refinement. The models undergo periodic re-training based on this feedback and predefined time windows, ensuring that they continually improve in accuracy and precision for future patients.

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

**Claims**

1. A system for oral cancer lesion diagnosis, comprising:

   an image capture device configured to capture at least one image of a mouth; and
   a platform comprising:

   an artificial intelligence feature including a machine learning model configured to detect a potential oral cancer lesion based on the at least one image of the mouth; and
   a user interface configured to receive user input.

2. The system of Claim 1, wherein the image capture device is further configured to capture at least one video of the mouth.

3. The system of Claim 1, wherein the user interface is configured to allow a user to manually mark and annotate the at least one image of the mouth.

4. The system of Claim 1, wherein the user interface includes at least one user role defining user access privileges to the platform.

5. The system of Claim 4, wherein the user roles include at least one of administrator, dentist, reviewer, data scientist, guest, and student.

6. The system of Claim 1, wherein the platform is configured to operate in an online mode and an offline mode.

7. The system of Claim 1, wherein the at least one image of the mouth is processed by the machine learning model.

8. The system of Claim 1, wherein upon detecting a potential oral cancer lesion, the artificial intelligence feature notifies the user.

9. The system of Claim 1, wherein upon detecting a potential oral cancer lesion, the artificial intelligence feature sends the at least one image of the mouth to a specialist for review.

10. The system of Claim 1, wherein the artificial intelligence feature further includes a segmentation model that processes the at least one image of the mouth and highlights a potential oral cancer lesion.

11. The system of Claim 10, wherein the artificial intelligence feature further includes a classification model.

12. The system of Claim 1, wherein the artificial intelligence feature is configured to perform pre-processing and transformation techniques including at least one of resizing the at least one image of the mouth, augmenting data associated with the at least one image of the mouth, and normalizing pixel values of the at least one image of the mouth.

13. A method for diagnosing an oral cancer lesion, comprising:

    providing an image capture device configured to capture at least one image of a mouth;
    providing a platform, the platform including an artificial intelligence feature including a machine learning model; and
    detecting a potential oral cancer lesion via the machine learning model based on the at least one image of the mouth.

14. The method of Claim 13, wherein the platform includes a user interface configured to receive user input.

15. The method of Claim 13, the method further comprising processing, via the machine learning model, the at least one image of the mouth.

16. The method of Claim 13, the method further comprising upon detecting a potential oral cancer lesion, notifying a user.

17. The method of Claim 13, the method further comprising upon detecting a potential oral cancer lesion, sending the at least one image of the mouth to a specialist for review.

18. The method of Claim 13, the method further comprising processing the at least one image of the mouth and highlighting a potential oral cancer lesion.

19. The method of Claim 13, wherein the artificial intelligence feature further includes a classification model.

20. The method of Claim 13, the method further comprising performing pre-processing and transformation techniques including at least one of resizing the at least one image of the mouth, augmenting data associated with the at least one image of the mouth, and normalizing pixel values of the at least one image of the mouth.

(a) Healthy    (b) Tongue Cheek Chewing    (c) Geographic Tongue

FIG. 1

FIG. 2

FIG. 3

FIG. 4

FIG. 5

FIG. 6

FIG. 7

FIG. 8

EP 4 679 447 A1

FIG. 9

FIG. 10

FIG. 11

DenTech Platform

FIG. 12

FIG. 13

FIG. 14

Europäisches
Patentamt

European
Patent Office

Office européen
des brevets

# EUROPEAN SEARCH REPORT

Application Number

EP 25 18 8882

## DOCUMENTS CONSIDERED TO BE RELEVANT

| Category | Citation of document with indication, where appropriate, of relevant passages | Relevant to claim |
|---|---|---|
| X | WO 2023/163670 A1 (T C ISTANBUL AYDIN UNIVERISTESI [TR]) 31 August 2023 (2023-08-31) * page 1 lines 9-11, p.5 l.31 - p.7 l.2, p.6 l.1-29, p.7 l.22-26 * ----- | 1-20 |
| X | US 2021/353216 A1 (HILLEN FLORIAN [US]) 18 November 2021 (2021-11-18) * paragraphs [0004], [0024], [0031], [0032] * ----- | 1-20 |
| X | US 2022/351500 A1 (BUNDSGAARD RICHARD [DK] ET AL) 3 November 2022 (2022-11-03) * paragraphs [0001], [0009] - [0010], [0012] - [0015], [0020], [0027] - [0033], [0083], [0156], [0200] * ----- | 1-20 |
| X | US 2022/189611 A1 (FARKASH SHAI [IL] ET AL) 16 June 2022 (2022-06-16) * paragraphs [0010], [0011], [0013], [0021], [0025], [0037], [0065], [0066], [0080] * ----- | 1-20 |

**CLASSIFICATION OF THE APPLICATION (IPC)**

INV.
G16H30/40
G16H50/20

**TECHNICAL FIELDS SEARCHED (IPC)**

G16H
A61B
G06V
G06T

The present search report has been drawn up for all claims

| Place of search | Date of completion of the search | Examiner |
|---|---|---|
| Munich | 26 November 2025 | Huber, Alexander |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.........................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 18 8882

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2025

| Patent document cited in search report | Publication date | Patent family member(s) | Publication date |
|---|---|---|---|
| WO 2023163670 A1 | 31-08-2023 | TR 2022002554 A2<br>WO 2023163670 A1 | 21-03-2022<br>31-08-2023 |
| US 2021353216 A1 | 18-11-2021 | US 2019313963 A1<br>US 2021353216 A1<br>US 2022192590 A1<br>US 2023371888 A1<br>WO 2019204520 A1 | 17-10-2019<br>18-11-2021<br>23-06-2022<br>23-11-2023<br>24-10-2019 |
| US 2022351500 A1 | 03-11-2022 | DK 201970623 A1<br>EP 4022648 A1<br>US 2022351500 A1<br>WO 2021064114 A1 | 01-07-2021<br>06-07-2022<br>03-11-2022<br>08-04-2021 |
| US 2022189611 A1 | 16-06-2022 | US 2022189611 A1<br>US 2024312605 A1 | 16-06-2022<br>19-09-2024 |

EPO FORM P0459

For more details about this annex : see Official Journal of the European Patent Office, No. 12/82

**EP 4 679 447 A1**

REFERENCES CITED IN THE DESCRIPTION

**Patent documents cited in the description**

- US 63670318 A